# EUROPEAN PATENT SPECIFICATION

(11) **EP 1 439 798 B1**
(45) Date of publication and mention of the grant of the patent: **05.10.2011**
(21) Application number: 02784138.6
(22) Date of filing: 17.10.2002
(51) Int. Cl.: A61F 2/01

(54) **DISTAL PROTECTION AND RETRIEVAL DEVICE**
DISTALE SCHUTZ- UND EINFANGSVORRICHTUNG
DISPOSITIF DE PROTECTION DISTALE ET DE RECUPERATION

(30) Priority: 29.10.2001 US 20629
(43) Date of publication of application: 28.07.2004
(73) Proprietor: Boston Scientific Limited, St. Michael (BB)
(72) Inventor: VRBA, Anthony, C., Maple Grove, MN 55311 (US); BEULKE, Mel, R., Bloomington, MN 55438 (US)
(74) Representative: Vossius & Partner
(86) International application number: PCT/US2002/033192
(87) International publication number: WO 2003/037218

(56) References cited:
- WO-A-01/80776
- WO-A-02/069845
- WO-A1-01/50982
- WO-A1-01/50982
- US-A- 6 152 946

## Description

Embodiments of this invention relate generally to the area of percutaneous intravascular medical devices and more particularly to distal protection devices.

There are a number of intravascular procedures where emboli may be created in the vasculature. It may be desirable to capture these emboli before they flow downstream. WO 01/50982 for instance refers to a vascular filter that includes a tubular member having proximal and distal ends and a guidewire lumen. By means of an inflatable member, the vascular filter may be frictionally engaged with the outer surface of the filter.

Embodiments of the invention pertain to a distal protection device adapted for remote deployment and later retrieval. The present invention refers to a distal protective system with a retired device and a distal protection device which includes a frame and filter material assembly for capturing emboli and which comprises a proximal portion having a ridge adapted for interface with a retrieval device. The distal protective device may be adapted for use with a guidewire. The distal protection device may be deployed distally of the site of the interventional procedure. The distal protective device may be deployed by sliding the device along a wire using a catheter or other wire until the desired location is reached. The distal protective device may be deployed from a sheath. When retrieval is desired, a balloon catheter may be slid distally over the wire until it reaches the device. The balloon may then be inflated to grip the device and the balloon catheter and the device may be withdrawn simultaneously.

Further examples are described in the following items 13 to 42 :
13. A distal protection system comprising:
   a filter cartridge having a proximal region with a ridge; and
   a catheter having a distally located inflatable cuff, the cuff having an inflated and a deflated position, wherein when the cuff is in the inflated position at a point relative to the filter cartridge where at least a portion of the cuff is distal the ridge, at least a portion of the filter cartridge is prevented from moving distally beyond the cuff.
14. The system of item 13, wherein the filter cartridge comprises a frame portion, a filter portion disposed on the frame portion, and a longitudinal portion having a proximal end, a distal end and a lumen disposed therebetween.
15. The system of item 13, wherein the retrieval device is comprised of a first longitudinal member having a proximal end, a distal end and a first lumen disposed therebetween, wherein the first lumen is fluidly connected to the cuff.
16. The system of item 13, wherein the retrieval device is further comprised of a second lumen disposed parallel to the first lumen, the second lumen having a proximal opening and a distal opening.
17. The system of item 13, wherein the proximal region with a ridge is a proximal section of the longitudinal portion.
18. The system of item 13, wherein the proximal region with a ridge is a proximal section of the frame portion.
19. The system of item 14, wherein the longitudinal portion has a substantially circular cross section.
20. The system of item 14, wherein the ridge is a distally decreasing region on outer perimeter of the proximal end of the longitudinal portion.
21. The system of item 14, wherein the longitudinal portion further comprises a second lumen having a proximal end and a distal end, wherein the second lumen is disposed around the first lumen, defining an inner longitudinal portion disposed between the first lumen and the second lumen and an outer longitudinal portion disposed around the second lumen, and wherein the outer longitudinal portion and the inner longitudinal portion are connected proximate the distal end of the outer longitudinal portion.
22. The system of item 21, wherein the ridge is a distally decreasing region on the outer perimeter of the inner longitudinal portion.
23. The system of item 21, wherein the ridge is a distally increasing region on the inner perimeter of the outer longitudinal portion.
24. The system of item 21, wherein the longitudinal member and the frame portion of the filter cartridge are monolithic.
25. The system of item 21, wherein the cuff in the expanded state has a toroidal shape.
26. The system of item 13, wherein the cuff comprises a plurality of expabandable balloons disposed at the distal end of the tube.
27. The system of item 13, wherein the cuff is disposed on the outer surface of the tube.
28. The system of item 13, wherein the cuff is disposed within the first lumen of the tube.
29. A method of retrieving a cartridge from a body vessel lumen, the method comprising the steps of:
   providing a filter cartridge disposed within a body vessel lumen;
   providing a retrieval device;
   advancing the retrieval device distally in the body vessel lumen until at least a portion of the retrieval device is distal the proximal most portion of the filter partridge;
   engaging the filter cartridge with the retrieval device such that at least a portion of the filter cartridge is prevented from moving distally beyond at least a distal portion of the retrieval device; and
   advancing the retrieval device and the filter cartridge proximally from the body lumen.
30. The method of item 29 wherein the retrieval device has a distally located inflatable cuff and the step of engaging the filter cartridge with the retrieval device includes expending the cuff.
31. The method of item 30 wherein the filter cartridge has a proximal ridge and wherein the step of advancing the retrieval device includes advancing the retrieval device until at least a portion of the inflatable cuff extends distally beyond the ridge.
32. The method of item 31 further comprising the steps of:
   providing a wire extending distally through at least a portion of the filter cartridge and proximally from the body lumen; and
   advancing the balloon catheter proximally over the wire.
33. The method of item 31 further comprising the steps of:
   advancing a sheath to a point proximate the distal end of the filter cartridge;
      and
   advancing the filter cartridge proximally into the sheath.
34. The method of item 31, wherein the step of providing a filter cartridge further comprises the steps of:
   advancing a wire distally to a filter cartridge deployment location;
   advancing a sheath distally over the wire; and
   advancing a filter cartridge through the sheath using a catheter.
35. A distal protection device comprising:
   A filter cartridge having a frame portion, a filter portion; and
   A proximal region having an area with an increased coefficient of friction for interface with a retrieval devices.
36. The device item 1, further comprising a radiopaque band on the proximal region.
37. The device of item 1, further comprising a second ridge on the proximal region.
38. The system of item 13, wherein the inflatable cuff has a first inflation chamber and a second inflation chamber located distal the first inflation chamber.
39. The system of item 13, further comprising a balloon on the catheter proximal the inflatable cuff.
40. The system of item 39, wherein the balloon is an angioplasty balloon.
41. The system of item 13, further comprising a radiopaque band proximate the inflatable cuff.
42. The system of item 13, wherein the inflatable cuff has a surface with an increased coefficient of friction.

Preferred embodiments of the invention will be discussed in the following with reference to the drawings, in which :
Figure 1. is a side view with a partial cross-section depicting a distal portion of an illustrative distal protection system 100 in a body vessel in accordance with the invention.
Figure 2 is a side view of a cross section of a more proximal portion of the illustrative distal protection system 100 in a body vessel in accordance with the invention.
Figure 3 is a side view with a partial cross-section depicting an illustrative distal protection system 200 in a body vessel in accordance with the invention.
Figure 4 is a side view with a partial cross-section depicting an illustrative distal protection system 300 in a body vessel in accordance with the invention.

### Detailed Description of the Invention

The following description should be read with reference to the drawings wherein like reference numerals indicate like elements throughout the several drawings. The detailed description and drawings represent select embodiments and are not intended to be limiting.

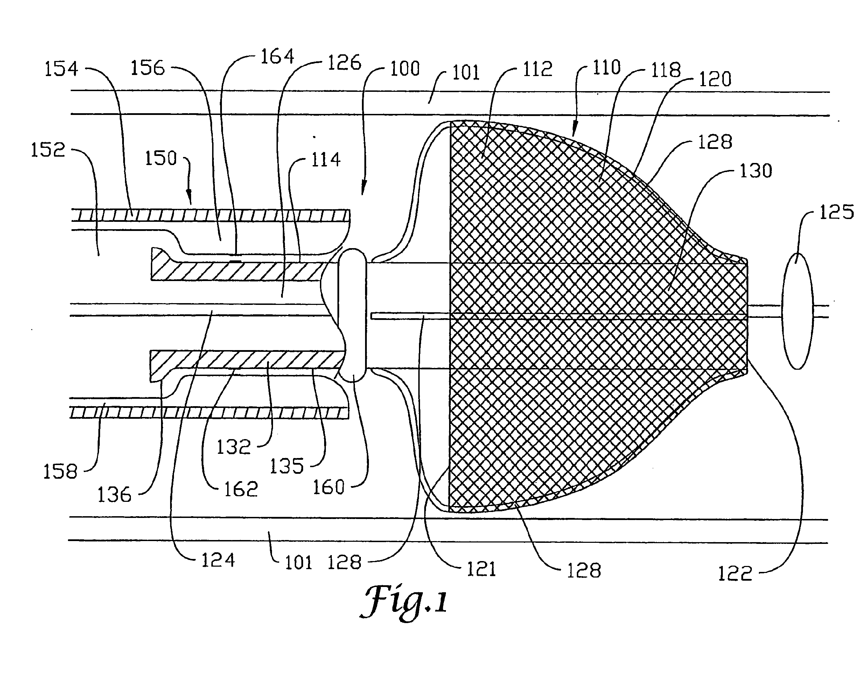

Figure 1 is a side view with a partial cross-section of an illustrative distal protection system 100 in accordance with the invention. Distal protection system 100 is shown in a body vessel lumen that includes vessel walls 101. Distal protection system 100 includes distal protection device 110 and a retrieval device 150. Distal protection device 110 is detachably coupled to retrieval device 150.

Distal protection device 110 includes a filter cartridge 112 and a proximal region 114. A lumen 126 is disposed between the proximal end of proximal region 114 and the distal end of filter cartridge 112 and is adapted for use with a guidewire 124. Filter cartridge 112 includes a filter material 118 disposed on and connected to a frame portion 120. Filter material 118, which may be substantially conical or hemispherical, includes an open area 121 adapted to substantially occlude the vessel lumen, and may include a opening 122 at the distal end of filter material 118 to allow passage of guidewire 124. Guidewire 124 may include a distally located stop 125.

In this figure, frame portion 120 is depicted as a plurality of struts 128 fixed at their proximal ends and distal ends on a longitudinal tube 130. Struts 128 are expandable in the middle and are sized to engage the wall 101 of the body vessel lumen when expanded. Struts 128 need not be attached to the proximal and distal ends of longitudinal tube 130; struts 128 may be attached to other portions of the tube.

Proximal region 114 is adapted for interface with a retrieval device and may be comprised of a longitudinal member 132. Distal protection device 110 may be adapted for interface with a retrieval device by having an outer perimeter 135 on longitudinal member 132 that has a distally decreasing diameter region 136. Distal protection device 110 may also be adapted by having an outer perimeter 135 on longitudinal member 132 which has increased friction. The increased friction may be created by adding a layer of material or by texturizing longitudinal member 132. Longitudinal member 132 may also include an area of increased diameter 160. This area of increased diameter may be used in order to aid in the positioning of the retrieval device or it may be used to aid in the deployment of the distal protection device. The area of increased diameter 160 may be monolithic with longitudinal member 132 or it may be a separate band of material. There may be a radiopaque band of material 162 on proximal region 114. This radiopaque band of material may be used to monitor the location of the distal protection device or it may be used to align the proximal region with the retrieval device.

Retrieval device 150 comprises a catheter 154 including a first lumen 152 adapted for use with a guidewire, a toroidal lumen 156 forming an inflatable cuff 156 disposed proximate the distal end of catheter 154, and a second lumen 158 fluidly connecting the proximal end of retrieval device 150 and inflatable cuff 156. Inflatable cuff 156 is designed to expand inwardly and is sized to slide distally over the outer perimeter of longitudinal member 132 when inflatable cuff 156 is in a contracted position. Inflatable cuff 156 may be sized to prevent inflatable cuff 156 from being slid proximally over distally decreasing region 136 when inflatable cuff 156 is inflated distal of distally decreasing region 136. Inflatable cuff 156 may also be sized to engage outer perimeter 135 of longitudinal member 132 when inflatable cuff 156 is in the expanded position. Inflatable cuff 156 may have an increased friction surface. This increased friction surface may be used to more securely engage the cuff on the distal protection device. Retrieval device 150 may include a radiopaque band of material 164. This radiopaque band of material 164 may be located on inflatable cuff 156 or it may be located on catheter 154. The band of material 164 may be used to located the end of retrieval device 150 or it may be used to align retrieval device 150 with the distal protection device.

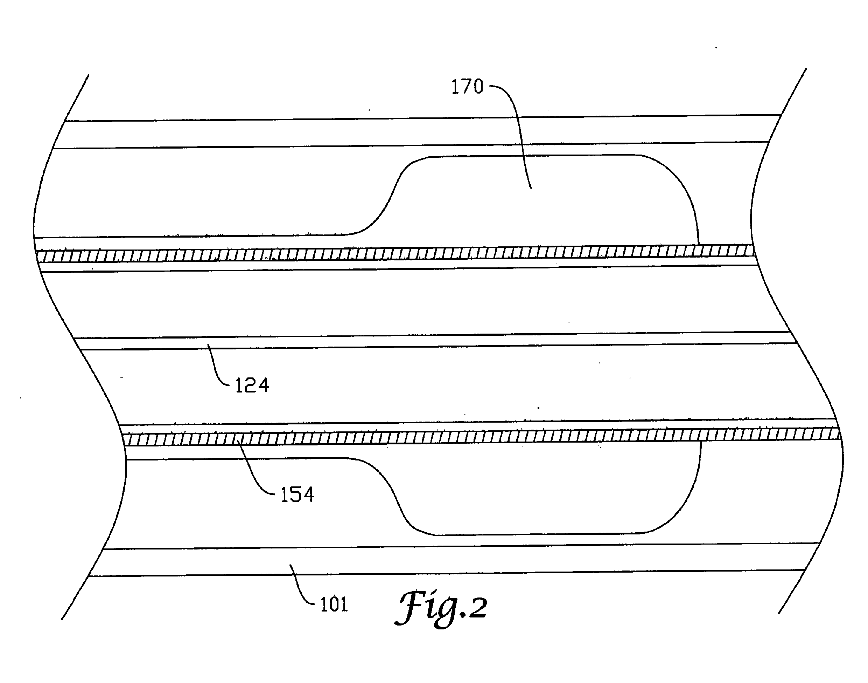

Turning to Figure 2, retrieval device 150 may also include a balloon 170 proximal of the inflatable cuff. This balloon 170 may be an angioplasty balloon, a stent delivery balloon or other balloon used in a medical procedure. Including this balloon on retrieval device 150 may reduce the number of device which need to be withdrawn and inserted during a medical procedure.

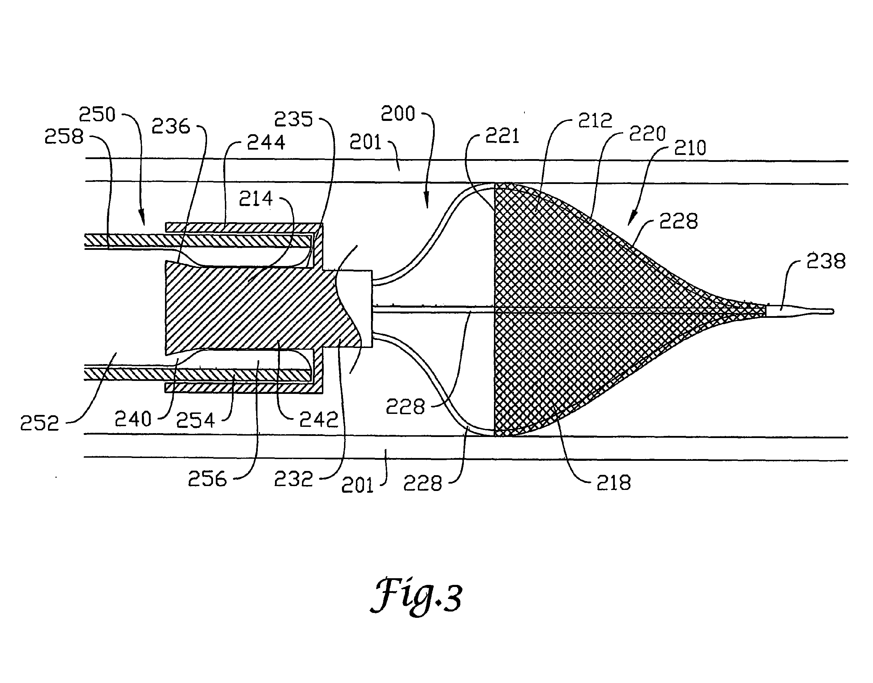

Figure 3 is a side view with a partial cross-section depicting an illustrative distal protection system 200 including distal protection device 210 in accordance with the invention. Distal protection system 200 is shown in a body vessel lumen which includes vessel walls 201. Distal protection system 200 comprises distal protection device 210 and retrieval device 250.

Distal protection device 210 may include a filter cartridge 212 and a proximal region 214. Filter cartridge 212 is comprised of a frame portion 220 joined distally to an atraumatic tip 238 and proximally to proximal region 214. Frame portion 212 includes a plurality of struts 228 and a filter material 218. Struts 228 are expandable and sized to engage the inner wall of the body vessel lumen (not depicted) when expanded. A filter material 218 is disposed on and connected to frame portion 220 and is substantially conical or hemispherical when frame portion 220 is expanded. Filter material is shaped to form an open area 221 adapted to substantially occlude the vessel lumen.

Proximal region 214 includes a longitudinal member 232 having a lumen 240, preferably a toroidal lumen 240 Lumen 240 may separate an inner portion 242 of longitudinal member 232 from an outer portion 244 of longitudinal member 232. Inner portion 242 and outer portion 244 are connected distally to each other. In this embodiment, longitudinal member 232 is adapted for interface with a retrieval device through inner portion 242 having an outer perimeter 235 which has a distally tapering region 236.

The filter cartridge and the proximal region are capable of many modifications and alternative forms. For example, the proximal region of Figure 3 is readily adapted for use with the filter cartridge shown in Figure 1 by including an appropriate lumen in inner portion 242. In another example, the proximal region of Figure 1 may be easily modified to have outer perimeter 135 of the inner portion be substantially constant and to have a distally increasing region on the inner perimeter of the outer portion. This modification would allow a retrieval device with an outwardly expanding cuff to be used to retrieve the distal protection device. In another example, the number of struts 228 may be increased to provide a more rounded open region 221 in filter material 218. Another modification may be the addition of a tapering region to the proximal end of proximal region 214 to facilitate the engagement with a retrieval device.

Retrieval device 250 comprises a first lumen 252 disposed between the proximal end and the distal end of a catheter 254, an inflatable cuff 256 disposed proximate the distal end of catheter 254, and a second lumen 258 fluidly connecting the proximal end of retrieval device 250 and inflatable cuff 256. Inflatable cuff 256 is designed to expand inwardly and is sized to slide distally over outer perimeter 235 of longitudinal member 232 when inflatable cuff 256 is in a contracted position. Inflatable cuff 256 maybe sized to prevent inflatable cuff 256 from being slid proximally over distally decreasing region 236 when inflatable cuff 256 is inflated distal to distally decreasing region 236. Inflatable cuff 256 may also be sized to engage the outer perimeter of longitudinal member 232 when inflatable cuff 256 is in the expanded position.

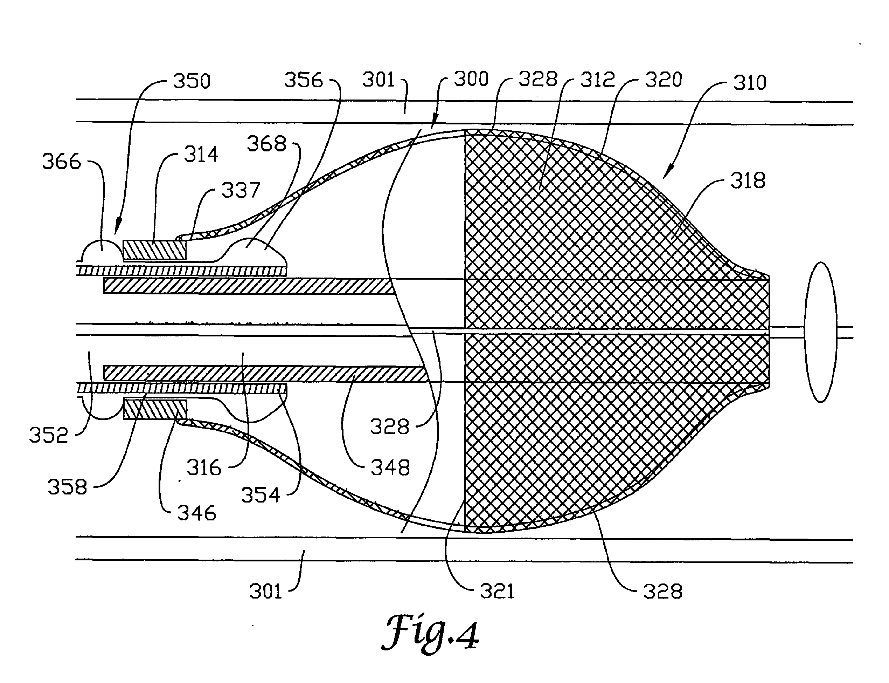

Figure 4 is a side view with a partial cross-section depicting an illustrative distal protection system 300 including distal protection device 310 in accordance with the invention. The distal protection system includes a retrieval device 350. Distal protection system 300 is shown in a vessel lumen which includes vessel walls 301.

Distal protection device 310 includes a proximal region 314 and a filter cartridge 312. In this embodiment, proximal region 314 and filter cartridge 312 are integral. Filter cartridge 312 is comprised of a frame portion 320, and a filter material 318.

Frame portion 320 generally comprises a plurality of struts 328 connected proximally to a ring 346 and distally to each other. An elongate member 348 may extend proximally from the distal end of struts 328. If use with a guidewire is desired, a lumen 316 may extend from the proximal end to the distal end of elongate member 348. Struts 328 are expandable in the middle and may be sized to engage the inner wall of the body vessel lumen when in the expanded state. Elongate member 348 may extend proximally to a point distal ring 346 or may extend to or beyond ring 346 proximally. Filter material 318 is disposed on and connected to struts 328 and ' includes an open area 321 designed to substantially occlude the vessel lumen. Proximal region 314 includes ring 346 and a proximal portion of the struts to create a distally increasing region 337 on the inner perimeter of the struts/ring portion of frame 320.

Retrieval device 350 comprises a first lumen 352 disposed between the proximal end and the distal end of a catheter 354, an inflatable cuff 356 disposed proximate the distal end of catheter 354, and a second lumen 358 fluidly connecting the proximal end of retrieval device 350 and inflatable cuff 356. Inflatable cuff 356 is designed to expand inwardly and is sized to slide distally under ring 346 when inflatable cuff 356 is in a contracted position. Inflatable cuff 366 may be sized to prevent inflatable cuff 356 from being slid proximally from distally increasing region 337 when inflatable cuff is inflated distal distally increasing region 337. Inflatable cuff 356 may optionally include a plurality of balloons arranged substantially equidistantly around the distal end of catheter 354. Inflatable cuff 356 may also have expansion chambers 366 and 368. These expansion chambers are adapted so that one may inflate distal ring 346 and one may inflate proximal ring 346. This prevents longitudinal movement of the distal protection device relative to the retrieval device.

Distal protection device 310 is susceptible to various changes and modifications. For example, ring 346 may have substantially the same inner and outer diameters as elongate member 348 with lumen 316. The ring would then be positioned proximal the elongate member leaving a gap between the distal end of the ring and the proximal end of the elongate member.

The construction of distal protection devices 110,210, or 310 is susceptible to many alternatives and modifications. Struts 128, 228, or 328 generally comprise substantially resilient biocompatible materials such as stainless steel and NiTi alloys. Filter material 118, 218, or 318 is selected to allow the flow of a fluid such as blood or radiographic contrast media while trapping emboli of a targeted size. Suitable filter materials may be a polyethylene mesh such as Saati Tech and Tetko, Inc. make. The filter material may be attached to the struts using sonic welding or adhesive. Longitudinal member 132, 232, or 348 may made from substantially flexible materials with some resistance to elastic deformation such as polyethylene, nylon, polyurethane, nickel titanium alloy, stainless steel, or silicone. Catheter 154,254, or 354 may be made from substantially flexible materials with good pushability and resistance to kinking such as polyethylene, polyethylene terephthalate, polybutylene terephthalate, polyether block amide, nylon, polyurethane with polyethylene, polyvinyl chloride, silicone, or NiTi alloys. Cuff 156, 256, or 356 may be made from a flexible material with good resistance to elastic deformation such as polyethylene terephthalate, polyether block amide, polybutylene terephthalate, a composite material with embedded filaments, or nylon. Ring 346 may be made from any of the materials that struts 328 or longitudinal member 348 may be made from.

A distal protection device may be deployed in a body vessel lumen using a wire or catheter which interfaces with or pushes on the device. If the distal protection device has a lumen extending through its length, it may be deployed on a guidewire. When retrieval of the distal protection device is desired, a retrieval device may be inserted distally until at least the distal portion of the retrieval device is distal the proximal most portion of the distal protection device. The retrieval device can then engage the distal protection device so that at least the proximal most portion of the distal protection device cannot move distally of the distal end of the retrieval device.
The engaging mechanism may be a pneumatically or hydraulically operated mechanism such as an inflatable cuff, it may be a mechanically operated mechanism such as a cable operated clamping mechanism, it may be an electrically operated actuation device, or it may be any mechanism able to engage the distal protection device. Once the retrieval device engages the distal protection device, both may be withdrawn proximally from the body vessel lumen, or the retrieval device may be used to position the distal protection device in a different place in the body vessel lumen.

In an example of a method of use of a distal protection device, the distal protection device such as distal protection device 110, 210, or 310 may be deployed in a body vessel lumen to a desired location. The distal protection device may be moved distally into and through the body vessel lumen using a tube or wire which pushes on or interfaces with the proximal end of the distal protection device. The catheter may also be similar to distal retrieval device 150, 250, or 350 in that it includes an inflatable cuff proximate the distal end to engage with the proximal end of the distal protection device. If the distal protection device has a lumen which extend through the length of the device, such as lumens 122,126, or 316, the distal protection device may be moved along a guidewire or other wire. The distal protection device may be compressed within a deployment sheath which may be moved distally to the desired location with the distal protection device or before the distal protection device is moved. When the distal protection device has reached the desired location, it may be moved distally from the deployment sheath or the deployment sheath may be moved proximally from it. When the device is moved from the deployment sheath, the struts expand and the filter material opening occludes the vessel lumen and begins the filtering process. The deployment sheath may then be withdrawn proximally.

If a guidewire is used, the guidewire may be withdrawn distally or may be left in place. The guidewire may include a stop distal of the distal protection device to prevent the device from moving further distally into the vessel lumen. The guidewire may also include a stop proximally to keep the distal protection device from moving proximally. If the distal protection device is moved distally over the guidewire, the proximal stop would be configured to allow passage of the device distally over the stop and prevent the device from moving proximally over the stop.

When retrieval of the distal protection device is desired, a retrieval device is deployed, such as retrieval device 150,250, or 350. If a guidewire is being used, the retrieval device may be moved distally along the guidewire until the inflatable cuff portion of the retrieval device is at least partially distal the ridge on the proximal portion of the distal protection device. If the distal protection device and the retrieval device include radiopaque bands, these bands may be used to align the devices. The inflatable cuff on the retrieval device is then inflated, thus preventing the distal protection device from being slid distal from the cuff. The retrieval device and the distal protection device may then be slid proximally from the body vessel lumen. If a retrieval sheath is used, the retrieval sheath is positioned proximate the proximal portion of the distal protection device. When the cuff is inflated, the distal protection device may then be moved into the lumen of the retrieval sheath. The frame portion of the distal protection device may then be compressed by the retrieval sheath. The retrieval device and the distal protection device may then be removed proximally from the body vessel lumen through the retrieval sheath or the retrieval device and the distal protection device may be removed proximally from the body vessel lumen together with the retrieval sheath.

The method of use described above is susceptible to many modifications and alternative forms. For example, after having withdrawn the distal protection device into a retrieval sheath using the retrieval device, the retrieval sheath may be moved distally with the distal protection device and the retrieval device to a new desired location. The retrieval device may then move the distal protection device distally from the retrieval sheath and the cuff may then be deflated, deploying the distal protection device at a new location.

While the invention is susceptible to various modifications, and alternative forms, specific examples thereof have been shown in the drawings and are herein described in detail. It should be understood, however, that the invention is not to be limited to the particular forms disclosed, but to the contrary, the invention is to cover all modifications, equivalents and alternatives falling within the scope of the appended claims.

## Claims

1. A distal protection system with a distal protection device and a retrieval device, the distal protection device comprising:
a filter cartridge (112, 212, 312) having a frame portion (120, 220, 320), a filter portion (118, 218, 318), and
a proximal region (114, 214, 314) having a ridge adapted for interface with the retrieval device (150, 250, 350), wherein
said retrieval device comprises an inflatable cuff (156, 256, 356) which may be inflated to grip at least a portion of the proximal region (114, 214, 314).

2. The system of claim 1, wherein the cuff (156, 256, 356) in the expanded state has a toroidal shape.

3. The system of claim 1, further comprising a longitudinal portion (132, 232, 348), wherein the longitudinal portion has a proximal end, a distal end and wherein the proximal region (114, 214, 314) is a proximal section of the longitudinal portion.

4. The system of claim 3, further comprising a lumen (126) between the proximal end and the distal end of the longitudinal portion (132, 232, 348).

5. The system of claim 1, wherein the proximal region (114, 214, 314) is a proximal section of the frame portion (120, 220, 320).

6. The system of claim 3, wherein the longitudinal portion (132, 232, 348) has a substantially circular cross section.

7. The system of claim 3, wherein the ridge is a distally decreasing region (136, 236) on the outer perimeter of the proximal end of the longitudinal portion.

8. The system of claim 4, wherein the ridge is a distally increasing region (337) on the inner perimeter of the proximal end of the longitudinal portion.

9. The system of claim 1, wherein the longitudinal portion (232) further comprises a toroidal lumen (240) having a proximal end and a distal end, wherein the toroidal lumen (240) defines an inner longitudinal portion (242) disposed within the toroidal lumen (240) and an outer longitudinal portion (244) disposed around the toroidal lumen (240), and wherein the outer longitudinal portion (244) and the inner longitudinal portion (242) are connected proximate the distal end of the outer longitudinal portion (232).

10. The system of claim 9, wherein the ridge is a distally decreasing region (236) on the outer perimeter of the inner longitudinal portion (242).

11. The system device of claim 9, wherein the ridge is a distally increasing region on the inner perimeter of the outer longitudinal portion.

12. The system device of claim 3, wherein the longitudinal portion (132, 232, 348) and the frame portion (120, 220, 320) of the filter cartridge are monolithic.

## Patentansprüche

1. Distales Schutzsystem mit einer distalen Schutzvorrichtung und einer Rückgewinnungsvorrichtung, wobei die distale Schutzvorrichtung aufweist:
eine Filterpatrone (112, 212, 312), die einen Rahmenabschnitt (120, 220, 320) und einen Filterabschnitt (118, 218, 318) aufweist, und
einen proximalen Bereich (114, 214, 314), der einen Grat aufweist, der zur Kopplung mit der Rückgewinnungsvorrichtung (150, 250, 350) eingerichtet ist, wobei die Rückgewinnungsvorrichtung eine aufblasbare Manschette (156, 256, 356) aufweist, die aufgeblasen werden kann, um mindestens einen Abschnitt des proximalen Bereichs (114, 214, 314) zu ergreifen.

2. System nach Anspruch 1, wobei die Manschette (156, 256, 356) im ausgedehnten Zustand eine toroidale Form aufweist.

3. System nach Anspruch 1, das ferner einen longitudinalen Abschnitt (132, 232, 348) aufweist, wobei der longitudinale Abschnitt ein proximales Ende und ein distales Ende aufweist, und wobei der proximale Bereich (114, 214, 314) ein proximaler Teil des longitudinalen Abschnitts ist.

4. System nach Anspruch 3, das ferner ein Lumen (126) zwischen dem proximalen Ende und dem distalen Ende des longitudinalen Abschnitts (132, 232, 348) aufweist.

5. System nach Anspruch 1, wobei der proximale Bereich (114, 214, 314) ein proximaler Teil des Rahmenabschnitts (120, 220, 320) ist.

6. System nach Anspruch 3, wobei der longitudinale Abschnitt (132, 232, 348) einen im Wesentlichen kreisförmigen Querschnitt aufweist.

7. System nach Anspruch 3, wobei der Grat ein distal abnehmender Bereich (136, 236) auf dem Außenumfang des proximalen Endes des longitudinalen Abschnitt ist.

8. System nach Anspruch 4, wobei der Grat ein distal abnehmender Bereich (337) auf dem Innenumfang des proximalen Endes des longitudinalen Abschnitts ist.

9. System nach Anspruch 1, wobei der longitudinale Abschnitt (232) ferner ein toroidales Lumen (240) mit einem proximalen Ende und einem distalen Ende aufweist, wobei das toroidale Lumen (240) einen inneren longitudinalen Abschnitt (242), der innerhalb des toroidalen Lumens (240) angeordnet ist, und einen äußeren longitudinalen Abschnitt (244) definiert, der um das toroidale Lumen (240) angeordnet ist, und wobei der äußere longitudinale Abschnitt (244) und der innere longitudinale Abschnitt (242) nahe des distalen Endes des äußeren longitudinalen Abschnitts (232) verbunden sind.

10. System nach Anspruch 9, wobei der Grat ein distal abnehmender Bereich (236) auf dem Außenumfang des inneren longitudinalen Abschnitts (242) ist.

11. System nach Anspruch 9, wobei der Grat ein distal abnehmender Bereich auf dem Innenumfang des äußeren longitudinalen Abschnitts ist.

12. System nach Anspruch 3, wobei der longitudinale Abschnitt (132, 232, 348) und der Rahmenabschnitt (120, 220, 320) der Filterpatrone monolithisch sind.

## Revendications

1. Système de protection distale avec un dispositif de protection distale et un dispositif de récupération, ledit dispositif de protection distale comprenant :
une cartouche filtrante (112, 212, 312) dotée d'une partie de cadre (120, 220, 320), une partie de filtre (118, 218, 318), et
une région proximale (114, 214, 314) dotée d'une arête prévue pour être en interface avec le dispositif de récupération (150, 250, 350), où
ledit dispositif de récupération comprend un ballonnet gonflable (156, 256, 356) pouvant être gonflé pour être en prise sur au moins une partie de la région proximale (114, 214, 314).

2. Système selon la revendication 1, où le ballonnet (156, 256, 356) a une forme toroïdale en état expansé.

3. Système selon la revendication 1, comprenant en outre une partie longitudinale (132, 232, 348), ladite partie longitudinale ayant une extrémité proximale, une extrémité distale et la région proximale (114, 214, 314) étant une section proximale de la partie longitudinale.

4. Système selon la revendication 3, comprenant en outre une lumière (126) entre l'extrémité proximale et l'extrémité distale de la partie longitudinale (132, 232, 348).

5. Système selon la revendication 1, où la région proximale (114, 214, 314) est une section proximale de la partie de cadre (120, 220, 320).

6. Système selon la revendication 3, où la partie longitudinale (132, 232, 348) présente une section transversale sensiblement circulaire.

7. Système selon la revendication 3, où l'arête est une région distalement décroissante (136, 236) sur le périmètre extérieur de l'extrémité proximale de la partie longitudinale.

8. Système selon la revendication 4, où l'arête est une région distalement croissante (337) sur le périmètre intérieur de l'extrémité proximale de la partie longitudinale.

9. Système selon la revendication 1, où la partie longitudinale (232) comprend en outre une lumière toroïdale (240) avec une extrémité proximale et une extrémité distale, ladite lumière toroïdale (240) définissant une partie longitudinale intérieure (242) disposée à l'intérieur de la lumière toroïdale (240) et une partie longitudinale extérieure (244) disposée autour de la lumière toroïdale (240), et la partie longitudinale extérieure (244) et la partie longitudinale intérieure (242) étant raccordées à proximité de l'extrémité distale de la partie longitudinale extérieure (232).

10. Système selon la revendication 9, où l'arête est une région distalement décroissante (236) sur le périmètre extérieur de la partie longitudinale intérieure (242).

11. Système selon la revendication 9, où l'arête est une région distalement croissante sur le périmètre intérieur de la partie longitudinale extérieure.

12. Système selon la revendication 3, où la partie longitudinale (132, 232, 348) et la partie de cadre (120, 220, 320) de la cartouche filtrante sont monolithiques.
